# EUROPÄISCHE PATENTSCHRIFT

(11) **EP 3 940 342 B1**
(45) Veröffentlichungstag und Bekanntmachung des Hinweises auf die Patenterteilung: **31.08.2022**
(21) Anmeldenummer: 20185402.3
(22) Anmeldetag: 13.07.2020
(51) Int. Cl.: G01D 3/036

(54) **MESSGERÄT**
MEASURING DEVICE
APPAREIL DE MESURE

(43) Veröffentlichungstag der Anmeldung: 19.01.2022
(73) Patentinhaber: Siemens Aktiengesellschaft, 80333 München (DE)
(72) Erfinder: Kirsammer, Florian, 76131 Karlsruhe (DE); Schmidt, Benjamin, 76187 Karlsruhe (DE)

(56) Entgegenhaltungen:
- EP-A1- 3 246 661
- EP-A1- 3 276 308
- DE-A1- 19 648 241

## Beschreibung

Die Erfindung betrifft ein erschütterungsempfindliches Messgerät
- mit einem Sensor, der in Abhängigkeit von einer erfassten Messgröße ein Sensorsignal erzeugt,
- mit einer Kompensationseinrichtung, die Erschütterungen des Messgerätes erfasst und ein Kompensationssignal erzeugt,
   und
- mit einer Auswerteeinrichtung, die ein Messergebnis aus einer Differenz zwischen dem Sensorsignal und dem Kompensationssignal erzeugt

Ein derartiges Messgerät ist aus der DE 27 02 978 A1 bekannt. Bei dem bekannten Messgerät handelt es sich um einen NDIR-Gasanalysator mit einem optopneumatischen Detektorsystem, das zur Detektion einer modulierten und ein Messgas durchstrahlenden Infrarot-Strahlung gasgefüllte und über eine Leitung mit einem darin angeordneten Strömungsfühler verbundene Detektorkammern aufweist. Zur Kompensation von Störungen des von dem Strömungsfühler erzeugten Messsignals aufgrund von pneumatischen Störeffekten in dem gasgefüllten Detektorsystem, die infolge der Trägheit des Gases bei Erschütterungen und/oder Beschleunigungen auftreten, ist eng benachbart zu dem Detektorsystem ein mit diesem weitgehend baugleiches gasgefülltes Kompensationssystem mit einem zweiten Strömungsfühler angeordnet. Das Kompensationssystem ist strahlungsundurchlässig abgeschlossen oder kann ein Gas enthalten, das im Infrarot-Bereich transparent ist, also kein Infrarot absorbiert. Die Signale des ersten Strömungsfühlers des Detektorsystems und des zweiten Strömungsfühlers des Kompensationssystems sind in Differenz geschaltet.

Da das Detektorsystem und das Kompensationssystem zwangsläufig voneinander beabstandet sind und bei Schwingungen oder Vibrationen neben translatorischen auch rotatorische Bewegungen auftreten können, kann es zu Phasenunterschieden zwischen den Ausgangssignalen des Detektorsystems und des Kompensationssystems kommen, so dass eine einfache Subtraktion des Ausgangssignals des Kompensationssystems von dem des Detektorsystems zu keinem befriedigenden Ergebnis führt und der Messfehler durch die angestrebte Kompensation sogar noch erhöht wird. Dies gilt auch bei toleranzbedingten baulichen Unterschieden zwischen dem Detektorsystem und dem Kompensationssystems und der elektrischen Verschaltung ihrer Sensoren.

Außerdem sind der bauliche Aufwand und die Kosten für das mit dem optopneumatischen Detektorsystem baugleiche Kompensationssystem erheblich.

MEMS Beschleunigungsaufnehmer und Gyroskope für die Erfassung von Bewegungen in Drohnen, Mobiltelefonen, Kraftfahrzeugen, Flugzeugen und mobilen IoT-Geräten sind allgemein bekannt. 3D-Beschleunigungsaufnehmer messen lineare Beschleunigungen entlang von drei orthogonalen Achsen, reagieren aber nicht auf Winkelgeschwindigkeiten. 3D-Gyroskope messen Winkelgeschwindigkeiten um die Achsen, reagieren aber nicht auf lineare Beschleunigungen. Mit der Technik der Sensorfusion können die Ergebnisse beider Sensoren kombiniert werden, um ein vollständiges und genaues Bild der Bewegung zu erhalten.

DE 196 48 241 A1, EP 3 246 661 A1 und EP 3 276 308 A1 beziehen sich ebenfalls auf Messgeräte mit einer Kompensationseinrichtung.

Der Erfindung liegt die Aufgabe zugrunde, die Störempfindlichkeit eines Messgeräts gegenüber Vibrationen und Erschütterungen, mit geringem gerätetechnischen Aufwand zu verringern.

Gemäß der Erfindung wird die Aufgabe durch das in Anspruch 1 angegebenen Messgerät gelöst, von dem vorteilhafte Weiterbildungen in den Unteransprüchen angegeben sind.

Gegenstand der Erfindung ist somit ein erschütterungsempfindliches Messgerät
- mit einem Sensor, der in Abhängigkeit von einer erfassten Messgröße ein Sensorsignal erzeugt,
- mit einer Kompensationseinrichtung, die Erschütterungen des Messgerätes erfasst und ein Kompensationssignal erzeugt, und
- mit einer Auswerteeinrichtung, die ein Messergebnis aus einer Differenz zwischen dem Sensorsignal und dem Kompensationssignal erzeugt,
   dadurch gekennzeichnet,
- dass die Kompensationseinrichtung eine mehrachsige MEMS inertiale Messeinheit mit einem Beschleunigungsaufnehmer allein oder zusammen mit einem Gyroskop enthält, die entsprechend der Anzahl der Achsen mehrere Bewegungssignale erzeugt, und
- dass die Kompensationseinrichtung einen Rechner mit einem Rechenmodell enthält, das dazu ausgebildet und trainiert ist, aus den Bewegungssignalen einen durch die Erschütterungen bedingten Störsignalanteil des Sensorsignals zu modellieren und als Kompensationssignal auszugeben, wobei das Rechenmodell derart trainiert ist, dass bei nichtvorhandener Messgröße die Differenz zwischen dem Sensorsignal und dem Kompensationssignal Null ist oder unter einer vorgegebenen Schwelle liegt.

Die mehrachsige MEMS inertiale Messeinheit bietet eine ausreichende Bandbreite und Auflösung, um die Erschütterungen innerhalb des Messgerätes zu erfassen. Aufgrund ihrer sehr geringen Baugröße kann die Messeinheit sehr nahe an dem Sensor positioniert werden, so dass bei Erschütterungen, Vibrationen o. dgl. die Messeinheit nahezu die gleiche Bewegung vollführt wie der Sensor. Unter den Bewegungssignalen sind die Beschleunigungssignale des Beschleunigungsaufnehmers und die Winkelgeschwindigkeitssignale des Gyroskops zu verstehen, wobei die Beschleunigungssignale durch Integration in Geschwindigkeitssignale umgewandelt werden können. Mittels des Rechenmodells werden die von der Messeinheit für die unterschiedlichen Drehachsen des Gyroskops und die Beschleunigungsachsen des Beschleunigungsaufnehmers gelieferten Bewegungssignale in den durch die Erschütterungen bedingten eindimensionalen Störsignalanteil des Sensorsignals umgerechnet.

Um die Abbildung der von der MEMS inertialen Messeinheit gelieferten Bewegungssignale auf die Beeinflussung des Sensorsignals zu lernen, kann das Messgerät vor seiner Inbetriebnahme, außerhalb des Messbetriebs oder in Messpausen, in denen der Sensor keine Messgröße erfasst, z. B. von der Messgröße abgeschirmt ist, gezielt mit Schwingungen oder Vibrationen angeregt werden, wobei die Abbildungsvorschrift derart angepasst wird, dass die Differenz zwischen dem Sensorsignal und dem berechneten Kompensationssignal Null ist oder unter einer vorgegebenen Schwelle liegt.

Der Lern- oder Trainingsvorgang kann für alle von der MEMS inertialen Messeinheit gelieferten Bewegungssignale gleichzeitig erfolgen. Dazu kann das Rechenmodell insbesondere ein neuronales Netz beinhalten, das die Bewegungssignale als Eingangsgrößen erhält und als Ausgangsgröße eine Schätzung des durch die Erschütterungen bedingten eindimensionalen Störsignalanteils des Sensorsignals erzeugt. Ein solches neuronales kann ggf. auch aus Teilnetzen, beispielsweise für die Bewegungssignale des Beschleunigungsaufnehmers und die des Gyroskops, bestehen, deren Ausgangsgrößen zusammengeführt werden.

Der rechnerische Aufwand für das Training der Rechenmodells kann reduziert werden, indem der erschütterungsbedingte Störsignalanteil des Sensorsignals in der Lern- oder Trainingsphase für jedes Bewegungssignal, d. h. für jede Beschleunigungsachse des Beschleunigungsaufnehmers und ggf. jede Drehachse des Gyroskops, separat gelernt wird. Dazu kann das Rechenmodell für jedes der Bewegungssignale jeweils ein digitales Filter enthalten, welches über die Einstellung seiner Filterkoeffizienten trainiert wird, einen durch die Erschütterungen bedingten und mit dem betreffenden Bewegungssignal korrelierenden Störsignalanteil des Sensorsignals zu modellieren. Im Messbetrieb des Messgeräts werden dann die Ausgangsignale der Filter zur Erzeugung des Kompensationssignals zusammengeführt, im einfachsten Fall addiert.

In der Regel wird das analoge Sensorsignal in einer Signalvorverarbeitungseinrichtung gefiltert und/oder verstärkt, bevor es digitalisiert und in einer digitalen Signalverarbeitungseinrichtung mittels einer Signalverarbeitungssoftware zu dem Messergebnis verarbeitet wird. Die digitalen Signalverarbeitungsschritte können z. B. eine digitale Filterung, eine Festlegung des Nullpunkts und eine Skalierung (Festlegung des Messbereichs) beinhalten. Das Messsignal ist oft von Störungen wie Netzbrummen, AC-Rauschen, DC-Drift usw. überlagert, die mittels Lock-In-Technik unterdrückt werden können, bevor das Messergebnis ermittelt wird (EP 2 130 295 A1). Dabei wird die Umwandlung der Messgröße in das analoge Sensorsignal mit einer Modulationsfrequenz moduliert und später das digitalisierte Sensorsignal durch Multiplikation mit einem Referenzsignal bei der Modulationsfrequenz phasensensitiv detektiert, um durch anschließende Tiefpassfilterung eine sog. Inphasenkomponente zu ermitteln, die direkt proportional zu dem eigentlichen Sensorsignal ist und zu dem Messergebnis verarbeitet wird. Da die Inphasenkomponente von der Phasendifferenz zwischen der Modulation und dem Referenzsignals abhängig ist, kann das modulierte digitale Sensorsignal zusätzlich mit dem um 90° phasenverschobenen Referenzsignal multipliziert und anschließend tiefpassgefiltert werden. Aus der so erhaltenen Quadraturkomponente und der Inphasenkomponente kann das Sensorsignal nicht-phasenbehaftet ermittelt werden (Zweiphasen-Lock-In-Technik).

Bei dem erfindungsgemäßen Messgerät durchläuft das Kompensationssignal parallel oder im Zeitmultiplex vorzugsweise dieselben digitalen Signalverarbeitungsschritte wie das Sensorsignal, wobei erst anschließend die Differenz zwischen den beiden verarbeiteten Signalen gebildet wird. Die Kompensation von erschütterungsbedingten Beeinträchtigungen des Sensorsignals erfolgt daher an dem durch die Signalverarbeitung von anderen Störungen wie Rauschen, Drift befreiten Sensorsignal, so dass das Kompensationssignal derartige Störungen nicht abbilden muss und dadurch die Berechnung des Kompensationssignals vereinfacht wird.

Nach dem gegenwärtigen Stand der Technik werden bei Mikroströmungsfühlern oder Wärmeleitfähigkeitsdetektoren, wie sie in der Gasanalytik eingesetzt werden, als Sensorelemente Metallfäden oder mäanderförmige Metallgitter verwendet, weil nur solche feinen Strukturen eine ausreichend geringe Wärmekapazität aufweisen und die geforderte Messempfindlichkeit ermöglichen. Von Nachteil ist jedoch die mechanische Empfindlichkeit von freihängenden und bei Aufheizung durchhängenden Metallfäden, was zum einen die Lebensdauer und zum anderen bei Vibrationen die Messgenauigkeit beeinträchtigt. Bei dem erfindungsgemäßen Messgerät kann es sich daher insbesondere um einen Gasanalysator strömungs- oder wechseldruckempfindlichen Sensorelement (Mikroströmungsfühler, Mikrophon) oder einem Wärmeleitfähigkeitsdetektor handeln.

Die erfindungsgemäße Kompensation erschütterungsbedingter Störsignalanteile in dem Sensorsignal ist unabhängig davon, welches Teil oder welche Komponenten des Messgerätes gegenüber Erschütterungen empfindlich sind. Erschütterungsempfindliche Teile oder Komponenten können z. B. der Chopper in einem NDIR-Gasanalysator oder optische Komponenten wie Spiegel im Strahlengang eines Gasanalysators sein. Entscheidend ist lediglich, dass sich die Erschütterungen auf das Sensorsignal auswirken.

Im Weiteren wird die Erfindung anhand von Ausführungsbeispielen und unter Bezugnahme auf die Figuren der Zeichnung erläutert; im Einzelnen zeigen:
- Fig. 1: ein erstes Beispiel für das erfindungsgemäße Messgerät,
- Fig. 2: ein weiteres Beispiel für das erfindungsgemäße Messgerät,
- Fig. 3: ein Messgerät in Form eines NDIR-Gasanalysators und
- Fig. 4: ein Messgerät in Form eines nach dem paramagnetischen Wechseldruckverfahren arbeitenden Gasanalysators.

Gleiche Bezugszeichen haben in den verschiedenen Figuren die gleiche Bedeutung. Die Darstellungen sind rein schematisch und repräsentieren keine Größenverhältnisse.

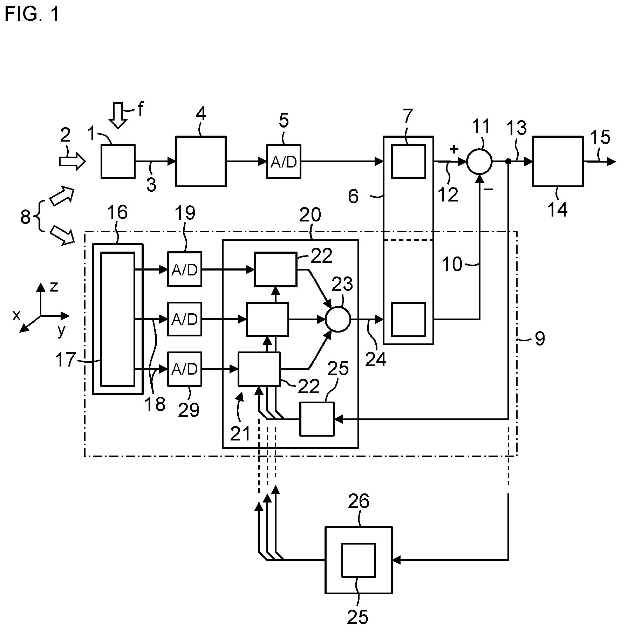

Fig. 1 zeigt ein Messgerät mit einem Sensor 1, der eine Messgröße 2 in ein analoges elektrisches Sensorsignal (Sensorrohsignal) 3 umwandelt. Das Sensorrohsignal 3 wird in einer analogen Signalvorverarbeitungseinrichtung 4 gefiltert und/oder verstärkt, bevor es in einem Analog-/Digital-Umsetzer 5 digitalisiert und in einer digitalen Signalverarbeitungseinrichtung 6 mittels einer Signalverarbeitungssoftware weiter verarbeitet, z. B. gefiltert, skaliert oder, wenn das Sensorsignal 3 mit einer Modulationsfrequenz f moduliert ist, mittels einer Lock-in-Demodulationseinrichtung 7 bei dieser Frequenz demoduliert wird.

Die Umwandlung der Messgröße 2 in das Sensorsignal 3 kann durch Erschütterungen oder Vibrationen 8 beeinflusst werden, die sich als Störsignalanteil in dem Sensorsignal 3 ausprägen. Daher wird mittels einer Kompensationseinrichtung 9 ein ein den Störsignalanteil schätzendes Kompensationssignal 10 erzeugt, das in einem Subtrahierer 11 von dem verarbeiteten digitalen Sensorsignal 12 subtrahiert wird. Aus der Differenz 13 beider Signale 10, 12 wird in einer Auswerteeinrichtung 14 ein Messergebnis 15, d. h. ein Messwert der Messgröße 2, ermittelt und ausgegeben.

Die Kompensationseinrichtung 9 enthält eine mehrachsige MEMS inertiale Messeinheit 16 mit einem 3-achsigen Beschleunigungsaufnehmer 17, der entsprechend der Anzahl der Achsen x, y, z drei unterschiedliche Bewegungssignale 18 erzeugt. Die MEMS inertiale Messeinheit 16 kann zusätzlich ein hier nicht gezeigtes 3-achsiges Gyroskop enthalten und in diesem Fall insgesamt sechs unterschiedliche Bewegungssignale erzeugen.

Der Beschleunigungsaufnehmer 17 misst lineare Beschleunigungen entlang der Achsen x, y, z und erzeugt dementsprechend die Bewegungssignale 18 in Form von Beschleunigungssignalen, die durch Integration in Geschwindigkeitssignale umgewandelt werden können (hier nicht gezeigt). Die Bewegungssignale 18 werden in Analog-/Digital-Umsetzern 19 digitalisiert und einem Rechner 20 mit einem Rechenmodell 21 zugeführt, das dazu dient, die Bewegungssignale 18 in den durch die Erschütterungen 8 bedingten eindimensionalen Störsignalanteil des Sensorsignals 3 umzurechnen bzw. diesen zu schätzen. Bei dem gezeigten Ausführungsbeispiel beinhaltet das Rechenmodell 21 für jedes der drei Bewegungssignale 18 jeweils ein digitales Filter 22. Die Ausgangssignale der Filter 22 werden in einem Summierer 23 zu einem Summensignal bzw. Ausgangssignal 24 des Rechenmodells 21 addiert, das in einem weiteren Kanal der hier 2-kanaligen Signalverarbeitungseinrichtung 6 dieselben Signalverarbeitungsschritte wie das digitalisierte Sensorsignal 3 durchläuft. Alternativ kann die Signalverarbeitungseinrichtung 6 einkanalig ausgebildet sein und die beiden Signale im Zeitmultiplex verarbeiten. Das Kompensationssignal 10 entspricht einer Schätzung des Störsignalanteils in dem verarbeiteten digitalen Sensorsignal 12.

Vor der Inbetriebnahme oder außerhalb des Messbetriebs, wenn der Sensor 1 keine Messgröße 2 erfasst, kann das Messgerät auf einem Schwingtisch nacheinander Vibrationen in Richtung jeder der drei Achsen x, y, z des Beschleunigungsaufnehmers 17 ausgesetzt werden. Indem die Vibrationsfrequenzen durchgestimmt werden, erhält man mit dem Signal 12 eine Übertragungsfunktion des Sensors 1 und mit dem Signal 10 eine Übertragungsfunktion der MEMS inertialen Messeinheit 16, beides Mal einschließlich der nachfolgenden Signalverarbeitung und separat für jede der drei Achsen x, y, z. Ergänzend kann während der Aufnahme der Übertragungsfunktionen für eine der drei Achsen, z. B. die x-Achse, immer nur das Ausgangssignal desjenigen Filter 22 dem Summierer 23 zugeführt werden, der das Bewegungssignal 18 der betreffenden Achse, hier also der x-Achse, erhält. Dazu können die den Filtern 22 zugeführten Bewegungssignale 18, die dem Summierer 23 zugeführten Ausgangssignale der Filter 22 oder die Filter 22 selbst einzeln ein- und ausgeschaltet werden. Mit Hilfe von Adaptionsalgorithmen 25 werden nacheinander in jedem der Filter 22 die Filterkoeffizienten derart und so lange verändert, bis die Differenz 13 zwischen den Signalen 12 und 10 minimal ist. Des geschieht, wie bereits erwähnt, nacheinander für jedes der drei Achsen x, y, z des Beschleunigungsaufnehmers 17 zugeordnetes Filter 22.

Mit entsprechend aufwendigen Adaptionsalgorithmen 25 ist es auch möglich, die Filtereinstellung für jeweils zwei oder alle drei Filter 22 gleichzeitig durchzuführen. Da die Einstellung der Filter 22 im Idealfall nur einmal, z. B. bei der Herstellung des Messgeräts erforderlich ist und ggf. nur in größeren Zeitabständen nachjustiert werden muss, können die Adaptionsalgorithmen 25 auch in einer externen Recheneinrichtung 26 gespeichert sein bzw. ausgeführt werden, wobei dann das Messgerät mit der externen Recheneinrichtung 26 verbunden wird.

Im Messbetreib des Messgeräts werden die Ausgangssignale der eingestellten Filter 22 in dem Summierer 23 addiert, wobei das Rechenmodell 21 zusammen mit der nachgeordneten Signalverarbeitung in der digitalen Signalverarbeitungseinrichtung 6 den erschütterungsbedingten Störsignalanteil des verarbeiteten digitalen Sensorsignals 12 in dem Kompensationssignal 10 abbildet. Daher werden durch die Differenzbildung beider Signale 10, 12 in dem Subtrahierer 11 erschütterungsbedingte Störsignalanteile in dem verarbeiteten digitalen Sensorsignals 12 beseitigt oder zumindest reduziert.

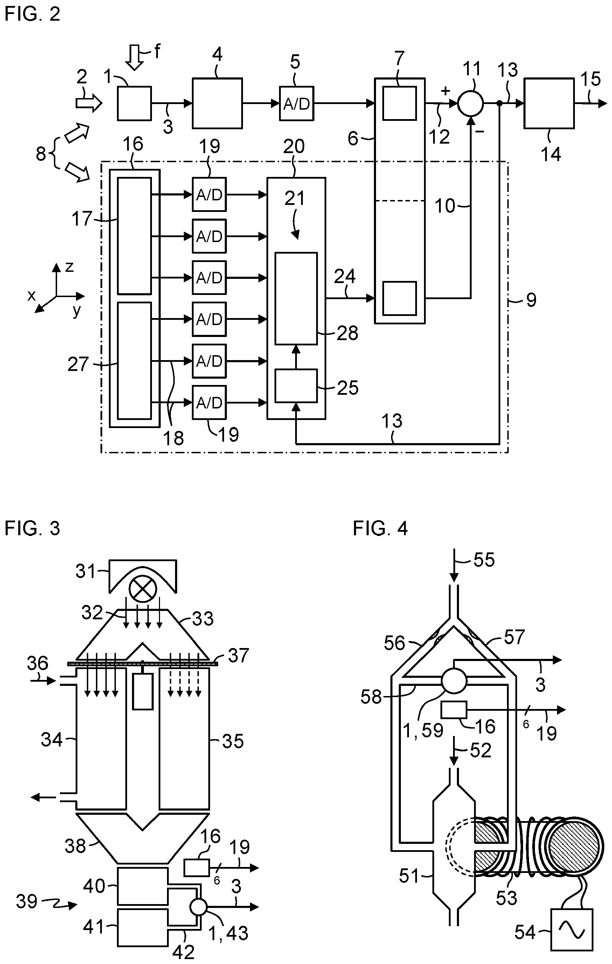

Fig. 2 zeigt ein weiteres Beispiel für das erfindungsgemäße Messgerät, das sich von dem nach Fig. 1 zunächst dadurch unterscheidet, dass die mehrachsige MEMS inertiale Messeinheit 16 zusätzlich zu dem 3-achsigen Beschleunigungsaufnehmer 17 ein 3-achsiges Gyroskop 27 enthält und entsprechend der Anzahl der Achsen x, y, z insgesamt sechs unterschiedliche Bewegungssignale 18 erzeugt. Während der Beschleunigungsaufnehmer 17 lineare Beschleunigungen entlang der Achsen x, y, z misst und dementsprechend Bewegungssignale 18 in Form von Beschleunigungssignalen erzeugt, misst das Gyroskop 18 Winkelgeschwindigkeiten um die Achsen x, y, z und erzeugt dementsprechend Bewegungssignale 18 in Form von Winkelgeschwindigkeitssignalen.

Das Rechenmodell 21 in dem Rechner 20 beinhaltet ein neuronales Netz 28, dem die digitalisierten Bewegungssignale 18 als Eingangsgrößen zugeführt werden. Das neuronale Netz 28 dient dazu, als Ausgangsgröße 24 eine Schätzung des durch die Erschütterungen 8 bedingten eindimensionalen Störsignalanteils des Sensorsignals 3 zu erzeugen. Dazu wird das neuronale Netz 28 vor der Inbetriebnahme des Messgerätes und/oder außerhalb des Messbetriebs oder in Messpausen wenn der Sensor 1 keine Messgröße 2 erfasst, mit Hilfe von Adaptionsalgorithmen 25 im Sinne einer Verringerung der Differenz 13 zwischen dem Kompensationssignal 10, also der Schätzung des Störsignalanteils in dem verarbeiteten digitalen Sensorsignal 12, und dem tatsächlichen gemessenen Störsignalanteil in dem verarbeiteten digitalen Sensorsignal 12 trainiert. Wenn die Differenz 13 zwischen beiden Signalen 10, 12 Null ist oder unter einer vorgegebenen Schwelle liegt, wird der erschütterungsbedingte Störsignalanteil des verarbeiteten digitalen Sensorsignals 12 am besten in dem Kompensationssignal 10 abgebildet.

Fig. 3 zeigt ein Messgerät in Form eines NDIR-Gasanalysators, hier z. B. in Zweistrahl-Ausführung, bei dem eine von einer Infrarot-Strahlungsquelle 31 erzeugte Infrarot-Strahlung 32 mittels eines Strahlteilers 33 auf einen Messstrahlengang durch eine Messküvette 34 und einen Vergleichsstrahlengang durch eine Referenzküvette 35 aufgeteilt wird. Die Messküvette 33 wird von einem Messgas 36 mit einer Messgaskomponente durchströmt, deren Konzentration zu bestimmen ist. Die Referenzküvette 35 ist mit einem Referenzgas, z. B. Stickstoff, gefüllt. Mittels einer zwischen dem Strahlteiler 33 und den Küvetten 34, 35 angeordneten Modulationseinrichtung 37, hier z. B. in Form eines rotierenden Blenden- oder Flügelrads, wird die Strahlung 32 abwechselnd durch die Messküvette 34 und Referenzküvette 35 freigegeben und gesperrt, so dass beide Küvetten abwechselnd durchstrahlt und abgeschattet werden. Die abwechselnd aus der Messküvette 34 und der Referenzküvette 35 austretende Strahlung wird mittels eines Strahlungssammlers 38 in ein optopneumatisches Detektorsystem 39 geleitet, das in bekannter Weise aus zwei hintereinander liegenden strahlungsdurchlässigen und mit der zu messenden Gaskomponente gefüllten Kammern 40, 41 besteht (Zweischichtdetektor), die über eine Leitung 42 mit dem darin angeordneten und das Messsignal 3 erzeugenden Sensor 1, z. B. einem Mikroströmungsfühler 43, verbunden sind. Das aufgrund der Differenz der Absorptionen in der Messküvette 34 und Referenzküvette 35 gebildete Messsignal 3 weist eine Modulationsfrequenz f auf.

In unmittelbarer Nähe zu dem optopneumatischen Detektorsystem 39 ist eine MEMS inertiale Messeinheit 16 angeordnet, deren Bewegungssignale 19 und das Sensorsignal 3 wie oben anhand von Fig. 1 beschrieben verarbeitet und bei der Frequenz f demoduliert werden. Da das Messsignal 3 neben der Modulationsfrequenz f auch einen aus der Summe der Absorptionen in der Messküvette 34 und Referenzküvette 35 bestehenden Störsignalanteil mit der zweifachen Modulationsfrequenz 2f enthält, kann zusätzlich eine phasensensitive Demodulation bei der doppelten Modulationsfrequenz 2f erfolgen, um aus der dabei erhaltenen Inphasenkomponente und Quadraturkomponente einen Diagnosewert zu ermitteln oder mit Hilfe eines solchen Diagnosewertes das Messergebnis zu normieren (EP 3 276 308 A1).

Fig. 3 zeigt beispielhaft das Messgerät in Form eines nach dem paramagnetischen Wechseldruckverfahren arbeitenden Gasanalysators. Dieser weist eine Messkammer 51 auf, die von einem Messgas 52, dessen Sauerstoffanteil bestimmt werden soll, durchströmt wird. Ein Teil der Messkammer 51 liegt zwischen den Polschuhen eines wechselstromgespeisten Elektromagneten 53 in dem von ihm erzeugten Magnetfeld. Der Elektromagnet 53 wird von einer Strom- oder Spannungsquelle 54 mit Wechselstrom angesteuert. Ein zur Erzielung des Messeffekts notwendiges Vergleichsgas 55 wird der Messkammer 51 durch zwei gleichförmige Kanäle 56, 57 zugeführt, wobei einer der beiden Vergleichsgasströme im Bereich des Magnetfeldes mit dem Messgas 52 zusammentrifft. Da Sauerstoffmoleküle aufgrund ihrer paramagnetischen Eigenschaft in dem Magnetfeld in Richtung höherer Feldstärke bewegt werden, entsteht zwischen den Vergleichsgasströmen in den Kanälen 56, 57 eine wechselnde Druckdifferenz mit der Frequenz f, die das Zweifache der Frequenz (1/2 f) des Wechselstroms ist. Diese bewirkt in einem Verbindungskanal 58 zwischen den beiden Kanälen 56, 57 eine Wechselströmung, die mittels des Sensors 1 hier in Form eines Mikroströmungsfühlers 59 erfasst und in das elektrische Messsignal 3 umgewandelt wird.

In unmittelbarer Nähe zu dem Mikroströmungsfühlers 59 ist eine MEMS inertiale Messeinheit 16 angeordnet, deren Bewegungssignale 19 und das Sensorsignal 3 wie oben anhand von Fig. 1 beschrieben verarbeitet und bei der Frequenz f demoduliert werden. Da dem Sensorsignal 3 mit der Frequenz f durch transformatorische Kopplung zwischen dem Elektromagneten 53 und dem Strömungsfühler 59 bzw. der nachgeordneten Signalverarbeitung ein Störsignalanteil mit der Frequenz 1/2 f des Wechselstroms sein kann, kann zusätzlich eine phasensensitive Demodulation bei der halben Modulationsfrequenz 1/2 f erfolgen, um aus der dabei erhaltenen Inphasenkomponente und Quadraturkomponente einen Diagnosewert zu ermitteln oder mit Hilfe eines solchen Diagnosewertes das Messergebnis zu normieren (EP 3 276 308 A1).

## Patentansprüche

1. Erschütterungsempfindliches Messgerät
- mit einem Sensor (1), der in Abhängigkeit von einer erfassten Messgröße (2) ein Sensorsignal (3) erzeugt,
- mit einer Kompensationseinrichtung (9), die Erschütterungen (8) des Messgerätes erfasst und ein Kompensationssignal (10) erzeugt, und
- mit einer Auswerteeinrichtung (14), die ein Messergebnis (15) aus einer Differenz (13) zwischen dem Sensorsignal (3, 12) und dem Kompensationssignal (10) erzeugt,
**dadurch gekennzeichnet,**
- **dass** die Kompensationseinrichtung (9) eine mehrachsige MEMS inertiale Messeinheit (16) mit einem Beschleunigungsaufnehmer (17) allein oder zusammen mit einem Gyroskop (27) enthält, die entsprechend der Anzahl der Achsen mehrere Bewegungssignale (18) erzeugt, und
- **dass** die Kompensationseinrichtung (9) einen Rechner (20) mit einem Rechenmodell (21) enthält, das dazu ausgebildet und trainiert ist, aus den Bewegungssignalen (18) einen durch die Erschütterungen (8) bedingten Störsignalanteil des Sensorsignals (3, 12) zu modellieren und als Kompensationssignal (10) auszugeben, wobei das Rechenmodell (21) derart trainiert ist, dass bei nichtvorhandener Messgröße (2) die Differenz (13) zwischen dem Sensorsignal (3, 12) und dem Kompensationssignal (10) Null ist oder unter einer vorgegebenen Schwelle liegt.

2. Messgerät nach Anspruch 1, **dadurch gekennzeichnet, dass** das Rechenmodell (21) ein neuronales Netz (28) beinhaltet.

3. Messgerät nach Anspruch 1, **dadurch gekennzeichnet,**
- **dass** das Rechenmodell (21) für jedes der Bewegungssignale (18) jeweils ein digitales Filter (22) mit einstellbaren Filterkoeffizienten enthält, das über die Einstellung seiner Filterkoeffizienten trainiert ist, einen durch die Erschütterungen (8) bedingten und mit dem betreffenden Bewegungssignal (18) korrelierenden Störsignalanteil des Sensorsignals (3, 12) zu modellieren, und
- **dass** das Rechenmodell (21) einen Summierer (23) enthält, der im Messbetrieb des Messgeräts die Ausgangsignale der Filter (22) zur Erzeugung des Kompensationssignals (10) addiert.

4. Messgerät nach einem der vorangehenden Ansprüche, **gekennzeichnet durch** eine Signalverarbeitungseinrichtung (6), in der das Sensorsignal (3) und das Kompensationssignal (10) vor Bildung der Differenz (13) jeweils gleich verarbeitet werden.

5. Messgerät nach Anspruch 4, **dadurch gekennzeichnet, dass** eine Modulationseinrichtung (33) vorhanden ist, die eine Modulation des Sensorsignals (3) mit einer Modulationsfrequenz (f) bewirkt, und dass die Signalverarbeitungseinrichtung (6, 24) eine Lock-in-Demodulationseinrichtung (7) enthält, die das Sensorsignal (3) und das Kompensationssignal (10) bei der Modulationsfrequenz (f) demoduliert.

6. Messgerät nach einem der vorangehenden Ansprüche, **dadurch gekennzeichnet, dass** es sich um einen Gasanalysator mit einem strömungs- oder wechseldruckempfindlichen Sensorelement (43, 59) oder einem Wärmeleitfähigkeitsdetektor handelt.

## Claims

1. Shock-sensitive measuring device
- having a sensor (1) that generates a sensor signal (3) in dependence upon a detected measured variable (2),
- having a compensating facility (9) that detects shocks (8) of the measuring device and generates a compensation signal (10), and
- having an evaluating facility (14) that generates a measurement result (15) from a difference (13) between the sensor signal (3, 12) and the compensation signal (10),
**characterised in that**
- the compensating facility (9) contains a multi-axis MEMS inertial measuring unit (16) having an acceleration sensor (17) alone or together with a gyroscope (27) and said measuring unit generates a plurality of movement signals (18) corresponding to the number of axes, and
- the compensating facility (9) contains a computer (20) having a computational model (21) that is embodied and trained for the purpose of modelling an unwanted signal portion of the sensor signal (3, 12), said unwanted signal portion arising as a result of the shocks (8), from the movement signals (18) and for the purpose of outputting said unwanted signal portion as a compensation signal (10), wherein the computational model (21) is trained in such a manner that in the absence of measured variables (2), the difference (13) between the sensor signal (3, 12) and the compensation signal (10) is zero or is below a predetermined threshold.

2. Measuring device according to claim 1, **characterised in that** the computational model (21) contains a neural network (28) .

3. Measuring device according to claim 1, **characterised in that**
- the computational model (21) for each of the movement signals (18) in each case contains a digital filter (22) that is trained via the setting of the filter coefficients of said digital filter to model an unwanted signal portion of the sensor signal (3, 12), said unwanted signal portion arising as a result of the shocks (8) and correlating with the relevant movement signal (18), and
- the computational model (21) contains a summing device (23) that adds the output signals of the filters (22) during the measuring operation of the measuring device so as to generate the compensation signal (10).

4. Measuring device according to one of the preceding claims, **characterised by** a signal processing facility (6) in which the sensor signal (3) and the compensation signal (10) are in each case processed identically prior to forming the difference (13).

5. Measuring device according to claim 4, **characterised in that** a modulation facility (33) is provided that triggers a modulation of the sensor signal (3) using a modulation frequency (f) and that the signal processing facility (6, 24) contains a lock-in demodulation facility (7) that demodulates the sensor signal (3) and the compensation signal (10) at the modulation frequency (f).

6. Measuring device according to one of the preceding claims, **characterised in that** said measuring device is a gas analyser having a flow-sensitive or alternating pressure-sensitive sensor element (43, 59) or a thermal conductivity detector.

## Revendications

1. Appareil de mesure sensible aux secousses
- comprenant un capteur (1), qui produit un signal (3) de capteur en fonction d'une grandeur (2) de mesure relevée,
- comprenant un dispositif (9) de compensation, qui détecte des secousses (8) de l'appareil de mesure et produit un signal (10) de compensation, et
- comprenant un dispositif (14) d'analyse, qui produit un résultat (15) de mesure, à partir d'une différence (13) entre le signal (3, 12) de capteur et le signal (10) de compensation,
**caractérisé**
- **en ce que** le dispositif (9) de compensation contient une unité (16) de mesure inertielle MEMS à plusieurs axes, ayant un enregistreur (17) d'accélération seul ou ensemble avec un gyroscope (27), qui produit plusieurs signaux (18) de mouvement en correspondance avec le nombre des axes, et
- **en ce que** le dispositif (9) de compensation contient un ordinateur (20) ayant un modèle (21) informatique, qui est constitué et qui a subi un apprentissage pour modéliser, à partir des signaux (18) de mouvement, une composante de signal perturbateur, due aux secousses (8), du signal (3, 12) de capteur et l'émettre comme signal (10) de compensation, le modèle (21) informatique ayant subi un apprentissage, de manière à ce que, pour des grandeurs (2) de mesure, qui ne sont pas présentes, la différence (13) entre le signal (3, 12) de capteur et le signal (10) de compensation soit nulle ou soit inférieure à un seuil donné à l'avance.

2. Appareil de mesure suivant la revendication 1, **caractérisé en ce que** le modèle (21) informatique comporte un réseau (28) neuronal.

3. Appareil de mesure suivant la revendication 1, **caractérisé**
- **en ce que** le modèle (21) informatique contient, pour chacun des signaux (18) de mouvement, respectivement un filtre (22) numérique ayant des coefficients de filtre réglables, qui subit un apprentissage par le réglage de ses coefficients de filtre, pour modéliser une composante de signal perturbateur, due aux secousses (8) et corrélée au signal (18) de mouvement concerné, du signal (3, 12) de capteur, et
- **en ce que** le modèle (21) informatique contient un sommateur (23) qui, dans le fonctionnement de mesure de l'appareil de mesure, additionne les signaux de sortie des filtres (22) pour la production du signal (10) de compensation.

4. Appareil de mesure suivant l'une des revendications précédentes, **caractérisé par** un dispositif (6) de traitement du signal, dans lequel le signal (3) de capteur et le signal (10) de compensation sont traités chacun de la même façon, avant la formation de la différence (13).

5. Appareil de mesure suivant la revendication 4, **caractérisé en ce qu'**il y a un dispositif (33) de modulation, qui provoque une modulation du signal (3) de capteur à une fréquence (f) de modulation, et **en ce que** le dispositif (6, 24) de traitement du signal contient un dispositif (7) de démodulation lock-in, qui démodule le signal (3) de capteur et le signal (10) de compensation à la fréquence (f) de modulation.

6. Appareil de mesure suivant l'une des revendications précédentes, **caractérisé en ce que** c'est un analyseur de gaz, ayant un élément (43, 59) capteur sensible à un écoulement ou à une pression changeante ou un détecteur de conductibilité de la chaleur.
